# EUROPÄISCHE PATENTANMELDUNG

(11) **EP 4 273 223 A1**
(43) Veröffentlichungstag der Anmeldung: **08.11.2023**
(21) Anmeldenummer: 23150459.8
(22) Anmeldetag: 05.01.2023
(51) Int. Cl.: C12N 1/20

(54) **VERFAHREN ZUR EXTRAKTION UND KONSERVIERUNG VON MIKROORGANISMEN AUS EINER STUHLPROBE EINES LEBEWESENS**

(30) Priorität: 04.05.2022 DE 102022111013
(71) Anmelder: Labor LS SE & Co. KG, 97708 Bad Bocklet-Großenbrach (DE)
(72) Erfinder: Balles, Jürgen, 97723 Oberthulba (DE); Rüffer, Andreas, 97708 Bad Bocklet (DE); Büttner, Christian, 96215 Lichtenfels (DE); Westhäuser, Florian, 96476 Bad Rodach-Rossfeld (DE)
(74) Vertreter: Paul & Albrecht Patentanwälte PartG mbB

(57) **Zusammenfassung**

Verfahren zur Extraktion und Konservierung von Mikroorganismen aus einer Stuhlprobe eines Lebewesens, insbesondere eines Menschen, umfassend die folgenden Schritte:
- Trennen einer Stuhlprobe in mehrere Fraktionen, wobei sich eine Mikroorganismen-Fraktion ausbildet, welche Mikroorganismen des Darmmikrobioms des Lebewesens enthält;
- Einfrieren der Mikroorganismen-Fraktion zur Konservierung derart, dass die Mikroorganismen zumindest teilweise in einer lebensfähigen Form erhalten bleiben.

## Beschreibung

Die vorliegende Erfindung betrifft ein Verfahren zur Extraktion und Konservierung von Mikroorganismen aus einer Stuhlprobe eines Lebewesens, insbesondere eines Menschen.

Im menschlichen Darm leben viele Billionen Bakterien. Mehrere Hundert verschiedene Spezies besiedeln insbesondere den Dickdarm und bilden das sogenannte Mikrobiom. Diese Bakterien sind nicht nur für das menschliche Immunsystem relevant, sondern auch für die Verdauung von Ballaststoffen verantwortlich. Sie produzieren Enzyme, welche diese für uns sonst unverwertbaren Nahrungsbestandteile aufspalten. Außerdem wehren sie Krankheitserreger ab und produzieren Vitamine.

Es ist bekannt, dass die Zusammensetzung des Mikrobioms von vielen unterschiedlichen Faktoren, unter anderem auch von der Lebensweise jedes einzelnen Menschen abhängt. Verschiebungen und Ungleichgewicht in der Zusammensetzung der Darmflora können Allergien, die Neigung zur Fettsucht und andere Krankheiten verursachen. Vor diesem Hintergrund ist eine gesunde, ausgewogene Darmflora wichtig für die Gesundheit eines jeden Menschen.

Insofern erfahren der Darm und seine Bakterienflora in den letzten Jahren ein immer größeres Interesse und zwar insbesondere im Rahmen der Beseitigung chronischer Darmentzündungen. Bei einem Befall des Darms mit Bakterieninfektionen, beispielsweise Entzündungen der Darmwand, die von den Giften des Bakteriums Clostridium-Difficile herrühren, erfolgt die Behandlung regelmäßig mit Antibiotika. Ein Problem besteht jedoch darin, dass die eingesetzten Antibiotika keinen Unterschied machen zwischen guten und für die Gesundheit unverzichtbaren Bakterien im Darm und denen, durch welche die zu behandelnde Entzündung verursacht wurde. Die Folge ist ein allgemeines Bakteriensterben im Darm der Patienten, und nicht selten breiten sich auf den freien Plätzen der abgestorbenen guten Bakterien die schädlichen Clostridien aus. Häufig folgt eine Entzündung des Darms, bis hin zur sogenannten pseudomembranösen Kolitis, mit einhergehenden hartnäckigen Durchfällen. Nach einer längeren Behandlung mit Antibiotika wird deshalb regelmäßig ein Neuaufbau der Darmflora medikamentös und/oder mit probiotischen Lebensmitteln begleitet.

Eine seit einigen Jahren eingesetzte alternative Behandlung von symptomatischen Infektionen mit Clostridium-Difficile, die seit Mitte 2016 auch in Deutschland zugelassen ist, besteht in einer Stuhltransplantation, bei welcher Stuhl von gesunden Spendern in den Darm von Kranken appliziert wird. Dabei wird die Darmflora von einer auf eine andere Person übertragen, was mit unterschiedlichen Begriffen bezeichnet wird. In der englischen Sprache wird häufig der Begriff "Fecal Microbiota Transplantation" verwendet.

Häufig wird eine solche Übertragung der Darmflora nach einer Behandlung mit Antibiotika vorgenommen. Der Ansatz besteht hierbei darin, dass eine geschädigte Darmflora Rückfälle stark begünstigt, da sich die entsprechenden Keime mittels mangelnder Konkurrenz physiologischer Darmkeime ungehindert vermehren können. Jedoch ist die Biodiversität des Stuhls bei einer Antibiotika-assoziierten Colitis im Vergleich zu normalen Stuhl deutlich reduziert.

Ein Problem bei der Stuhltransplantation eines fremden Spenders besteht darin, dass ein geeigneter Spender zur Verfügung stehen muss. Zum Teil ist es möglich, den Stuhl eines Spenders unmittelbar vor der Transplantation zu gewinnen und für die Transplantation vorzubereiten. Dies gilt insbesondere, wenn beispielsweise der Stuhl von den Kindern eines Patienten stammt. Dies ist jedoch nicht immer möglich. Problematisch ist, dass häufig zwischen der Entnahme und dem Einsetzen ein längerer Zeitraum besteht. Jedoch ist eine längere Lagerung des Stuhls bisher schwierig, da sich die darin enthaltenden Mikroorganismen verändern können.

Ferner existieren neuere Bestrebungen, wonach eine Eigenstuhltransplantation durchgeführt wird. Das bedeutet, dass vor einer Behandlung mit Antibiotika dem Patienten Stuhlproben entnommen werden, die dann längerfristig aufbewahrt werden, um die darin enthaltenden Mikroorganismen nach einer Antibiotikabehandlung in den Darm des Patienten einzusetzen. Auch bei einer solchen Vorgehensweise besteht die Notwendigkeit, die im Stuhl enthaltenen Mikroorganismen längerfristig einlagern zu können.

Vor diesem Hintergrund liegt die Aufgabe der folgenden Erfindung darin, ein Verfahren anzugeben, mit dem der Stuhl eines Spenders für eine Transplantation auch längerfristig eingelagert werden kann.

Erfindungsgemäß ist diese Aufgabe gelöst durch ein Verfahren der eingangs genannten Art, welches die folgenden Schritte umfasst:
- Trennen einer Stuhlprobe in mehrere Fraktionen, wobei sich eine Mikroorganismen-Fraktion ausbildet, welche Mikroorganismen des Darmmikrobioms des Lebewesens enthält;
- Einfrieren der Mikroorganismen-Fraktion zur Konservierung derart, dass die Mikroorganismen zumindest teilweise in einer lebensfähigen Form erhalten bleiben.

Der Erfindung liegt damit die Überlegung zugrunde, eine Stuhlprobe in mehrere Fraktionen zu trennen, wobei sich eine Fraktion ausbildet, in der die relevanten Mikroorganismen konzentriert enthalten sind. Anschließend ist vorgesehen, diese Fraktion einzufrieren, gegebenenfalls mit weiteren Zusatzstoffen, wobei sichergestellt wird, dass die Mikroorganismen zumindest teilweise in einer lebensfähigen Form erhalten bleiben. Insbesondere sollen die Mikroorganismen dabei einen intakten Zellstatus behalten, so dass sie auch nach einer längeren Lagerung in den Körper eines Patienten eingesetzt werden und ihre Funktion ausüben können. Prinzipiell kann die Mikroorganismen-Fraktion in den Körper des Spenders eingesetzt werden oder in den Körper eines anderen Patienten.

In weiterer Ausgestaltung des erfindungsgemäßen Verfahrens kann vorgesehen sein, dass das Trennen der Stuhlprobe in mehrere Fraktionen mittels Dichtegradientenzentrifugation erfolgt. Dabei handelt es sich um eine physikalische Trennmethode, bei der die unterschiedliche Dichte verschiedener Probenbestandteile zur Trennung genutzt wird.

Bevorzugt wird vor dem Trennen der Stuhlprobe in mehrere Fraktionen eine Hilfssubstanz mit einem präformierten Dichtegradienten in einem Behälter hergestellt. Unter einem präformierten Dichtegradienten ist zu verstehen, dass die Dichte der Hilfssubstanz in Richtung des Behälterbodens kontinuierlich zunimmt. Konkret kann die Hilfssubstanz ein chemisch inertes Mittel, insbesondere Cäsiumchlorid, Percoll, Saccharose und/oder lodixanol enthalten. Solche Mittel zeichnen sich dadurch aus, dass sie unter bestimmten Bedingungen einen Dichtegradienten in einem Behälter ausbilden können.

Bevorzugt enthält die Hilfssubstans lodixanol, welches mit Ringerlösung verdünnt ist. insbesondere kann es sich dabei um ¼ -Ringerlösung (autoklaviert) handeln. Die Verwendung von lodixanol hat dabei nicht nur den Vorteil, dass dieses Mittel geeignet ist, einen Dichtegradienten auszubilden. Vielmehr wirkt lodixanol auch als ein sogenannter Kryoschutzstoff, was bedeutet, dass er in Verbindung mit den Mikroorganismen beim späteren Einfrieren die Ausbildung von großen Eiskristallen verhindert. Solche Eiskristalle könnten die Zellmembranen der zu erhaltenden Mikroorganismen durchdringen und irreparabel beschädigen. Somit überstehen die Mikroorganismen das anschließende Einfrieren und Lagern weitgehend unbeschadet, da der intakte Zellstatus der Mikroorganismen durch die Anwesenheit von lodixanol aufrechterhalten wird.

In weiterer Ausgestaltung kann die Konzentration an lodixanol in der Hilfssubstanz mindestens 10 Gewichts-%, insbesondere mindestens 20 Gewichts-%, und/oder höchstens 40 Gewichts-%, insbesondere höchstens 30 Gewichts-%, bevorzugt 25 Gewichts-% betragen.

Um einen präformierten Dichtegradienten in der Hilfssubstanz zu erzeugen, kann die Hilfssubstanz eingefroren und anschließend aufgetaut werden. Konkret kann die Hilfssubstanz bis zu einer Temperatur von mindestens -15°C, insbesondere von -20°C eingefroren werden und/oder anschließend bei einer Umgebungstemperatur von mindestens 2°C, insbesondere von 4°C aufgetaut werden. Das Einfrieren und Auftauen kann jeweils über einen Zeitraum von etwa 8 bis 24 Stunden erfolgen. Auf diese Weise wird ein präformierter Dichtegradient in der Hilfssubstanz ausgebildet.

Ferner kann vor dem Trennen der Stuhlprobe in mehrere Fraktionen die Stuhlprobe insbesondere mit Ringerlösung verdünnt und/oder homogenisiert werden, sodass eine Stuhlsuspension entsteht. Durch das Verdünnen bzw. Homogenisieren der Stuhlprobe wird diese in einen flüssigeren Zustand versetzt, so dass sie bei der anschließenden Zentrifugation mit der Hilfssubstanz sich einfacher in verschiedene Fraktionen trennen lässt.

Konkret kann zur Dichtegradientenzentrifugation die Stuhlsuspension gemeinsam mit der Hilfssusbstanz mit dem präformierten Dichtegradienten in einem Behälter zentrifugiert werden, sodass sich in dem Behälter verschiedene schichtförmige Fraktionen ausbilden. Während der Zentrifugation sedimentieren die verschiedenen Fraktionen mit unterschiedlicher Geschwindigkeit so lange, bis die Dichte der Hilfssubstanz höher ist als die individuelle Dichte der verschiedenen Bestandteile. Konkret stellt sich dabei ein Gleichgewicht zwischen den Vorgängen der Sedimentation und der Diffusion ein, was zur Bildung von definierten Fraktionen führt. Diese finden sich als Schichten in dem Behälter wieder. Bevorzugt liegt nach der Dichegradientenzentrifugation etwa in der Mitte des Behälters eine Mikroorganismen-Fraktion vor, wobei sich oberhalb ein Überstand und unterhalb ein Rest der Stuhlprobe befindet.

Anschließend kann die Mikroorganismen-Fraktion von den übrigen Fraktionen getrennt werden. Dies kann dadurch erfolgen, dass schrittweise zunächst ein Überstand mit einer entsprechenden Pipette dem Behälter entnommen wird. Anschließend kann die Mikroorganismen-Fraktion ebenfalls mit einer Pipette entnommen werden und in ein separates Gefäß überführt werden. An der Behälterwandung haftende Mikroorganismen können mit einer entsprechenden Impföse mechanisch abgelöst und resuspendiert werden.

Die Zentrifugation erfolgt dabei bevorzugt bei mindestens 5.000 g, insbesondere mindestens 10.000 g, bevorzugt bei 15.000 g für eine Dauer von mindestens 10 Minuten, insbesondere mindestens 30 Minuten, bevorzugt für eine Dauer von 60 Minuten. Die Temperatur während der Zentrifugation kann mindestens 1°C, bevorzugt mindestens 2°C, und/oder höchstens 10°C, insbesondere höchstens 5°C betragen. Um die während der Zentrifugation aufgetrennten Fraktionen nicht zu vermischen, wird die Zentrifugation zweckmäßigerweise ungebremst beendet.

Vor dem Einfrieren der Mikroorganismen-Fraktion kann die Zellzahl und/oder der Zellstatus der Mikroorganismen in der Mikroorganismen-Fraktion ermittelt werden. Dieser Ausgestaltung des Verfahrens liegt die Überlegung zugrunde, dass auf diese Weise die Zellen, das heißt die Mikroorganismen, gezählt und hinsichtlich ihrer molekularen und physikalischen Eigenschaften analysiert werden können. Auf diese Weise kann die Qualität der entnommenen Mikroorganismen-Fraktion beurteilt werden, was für das spätere Einsetzen in den Körper eines Patienten relevant ist.

Konkret kann die Bestimmung der Zellzahl und/oder des Zellstatus mittels Durchflusszytrometrie erfolgen. Dabei werden die einzelnen Mikroorganismen durch hydrodynamische Fokussierung vereinzelt und an verschiedenen Lasern vorbeigeführt. Sobald ein Laserstrahl auf den betreffenden Mikroorganismus trifft, streut dieser das einfallende Licht. Dabei wird differenziert zwischen einer Vorwärtsstreuung (Forward Scatter, FSC) und einer Seitwärtsstreuung (Side Scatter, SSC). Die Vorwärtsstreuung liefert dabei eine Aussage über die Größe des Mikroorganismus, wohingegen die Seitwärtsstreuung Auskunft über deren Granularität gibt.

Zur Durchführung der Durchflusszytrometrie kann eine Probe der Mikroorganismen-Fraktion bis zur Verdünnungsstufe 10⁻⁵ verdünnt werden, wobei insbesondere die Verdünnung mit Ringerlösung erfolgt. Nach der Verdünnung kann der Probe eine Arbeitslösung, welche Fluoreszenzfarbstoffe enthält, zugeführt werden. Durch die Verwendung entsprechender Fluoreszenzfarbstoffe können bei der Durchflusszytometrie zusätzliche Informationen über den betreffenden Mikroorganismus gewonnen werden.

Konkret kann die Arbeitslösung die Fluoreszenzfarbstoffe SYBR Green I und Propidiumiodid enthalten. Dieser Ausgestaltung des Verfahrens liegt die Überlegung zugrunde, dass sich SYBR in die DNA von Mikroorganismen mit intakter, aber auch permeabler Zellmembran einlagern kann, wohingegen Propidiumiodid intakte Zellmembranen nicht durchdringen kann und sich dementsprechend nur in die DNA von Mikroorganismen mit geschädigter oder zerstörter Zellmembran einlagern kann. Abhängig von der Intensität der beiden Fluoreszenzfarbstoffe können so die Zellen hinsichtlich des Zustands ihrer Zellmembran differenziert werden. Dabei werden die Zellen in der Regel in den Zellstatus "lebend", "tot" oder "geschädigt" eingeteilt. Bei Zellen mit einem lebenden Zellstatus weist die Streuung des Farbstoffs SYBR eine hohe Intensität auf, wohingegen die Streuung von Propidiumiodid eine sehr geringe Intensität aufweist. Bei einem Zellstatus "tot" zeigen beide Fluoreszenzfarbstoffe eine hohe Intensität, bei einem geschädigten Zellstatus beide eine mittlere Intensität. Auf diese Weise können die einzelnen Mikroorganismen in Hinblick auf ihren Zellstatus analysiert werden.

Generell kann bei der Durchflusszytometrie die Vorwärtsstreuung und die Seitwärtsstreuung von einem auf die Probe auftreffenden Laserstrahl erfasst werden, um die Zellzahl und den Zellstatus der Mikroorganismen in der Probe zu ermitteln.

Das Einfrieren der Mikroorganismen-Fraktion kann auf eine Temperatur unterhalb von -50°C, insbesondere von unterhalb von -60°C, bevorzugt auf eine Temperatur von -80°C erfolgen. Dabei kann die Abkühlrate mindestens 0,1°C pro Minute, insbesondere mindestens -0,5°C pro Minute, und/oder höchstens -5°C pro Minute, insbesondere höchstens -2,5°C pro Minute, bevorzugt -1°C pro Minute betragen. Ein derartiges Abkühlen ermöglicht eine langfristige Konservierung, wobei die Mikroorganismen überwiegend das Einfrieren und die Lagerung unbeschadet überstehen. Insbesondere wenn lodixanol in der Hilfssubstanz enthalten ist und somit beim Zentrifugieren in Kontakt mit der Stuhlprobe bzw. der Stuhlsuspension kommt, wirkt es als ein Kryoschutzstoff und verhindert Beschädigungen an den Mikroorganismen beim Einfrieren.

Damit können mit dem zuvor beschriebenen Verfahren Stuhlproben einem Patienten entnommen werden, wobei die Mikroorganismen des Mikrobioms des Darms extrahiert und eingelagert werden, bevor sie dem gleichen oder einem anderen Patienten wiedereingesetzt werden.

Für die weitere Ausgestaltung des erfindungsgemäßen Verfahrens wird auf die Unteransprüche und auf die nachstehende Beschreibung eines Ausführungsbeispiels verwiesen.

Zur Durchführung des erfindungsgemäßen Verfahren zur Extraktion und Konservierung von Mikroorgansimsen aus einer Stuhlprobe wird in diesem Ausführungsbeispiel zunächst eine lodixanol-Hilfssubstanz mit einer Konzentration von 25 Gewichts-% hergestellt. Dazu wird die Lösung OptiPrep^{™} der PROGEN Biotechnik GmbH, welche 60 Gewichts-% lodixanol enthält, mit ¼ Ringerlösung (autoklaviert) verdünnt. Anschließend werden 75 ml dieser lodixanol-Hilfssubstanz in ein 225 ml-Zentrifugengefäß gegeben und bei -20°C über Nacht, das heißt über etwa 10 bis 12 Stunden, eingefroren. Anschließendes Auftauen über einen ähnlichen Zeitraum bei 4°C in einem Kühlschrank führt zu der Ausbildung eines kontinuierlichen Dichtegradienten in dem Zentrifugengefäß.

Ferner wird zur Vorbereitung 50 g Stuhl auf 0,1 g genau eingewogen und in einen Filterbeutel eingebracht. Nach weiterer Zugabe von etwa 150 ml Ringerlösung (autoklaviert) in den Filterbeutel wird der Filterbeutel 2 cm unterhalb der Öffnung mit einem entsprechenden Clip verschlossen. Anschließend kann die Probe in einem Mixer für eine Dauer von etwa 5 Minuten und einem Paddelabstand von 3 mm homogenisiert werden. Die dabei entstehende Stuhlsuspension wird aus der Tasche des Filterbeutels mittels einer 100ml-serologischen Pipette entnommen, wobei gleichzeitig das Volumen bestimmt wird und in ein 225 ml-Zentrifugengefäß überführt. Falls weniger als 150 ml Volumen vorliegt, kann die entsprechende Menge durch Zugabe von ¼ Ringerlösung (autoklaviert) auf 150ml aufgefüllt werden.

Anschließend wird die Stuhlsuspension unter Neigung des 225 ml- Zentrifugengefäßes mittels einer 50 ml-serologischen Pipette der Hilfssubstanz mit dem präformierten Dichtegradienten beigefügt. Danach wird das auf diese befüllte Zentrifugengefäß bei 15.000 g für eine Dauer von 60 Minuten und bei einer Temperatur von 4°C zentrifugiert. Die Beschleunigung der Zentrifuge erfolgt dabei auf höchster Stufe, während die Zentrifugation ungebremst beendet wird, damit sich die verschiedenen Fraktionen nicht vermischen.

Durch diese Dichtegradientenzentrifugation bilden sich in dem Zentrifugengefäß mehrere Fraktionen, wobei sich eine Mikroorganismen-Fraktion etwa in der Mitte des Gefäßes ausbildet, welche Mikroorganismen des Darmmikrobioms des Lebewesens in konzentrierter Form enthält.

Danach wird zunächst der Überstand, welcher sich über der Mikroorganismen-Fraktion in dem Zentrifugengefäß befindet, abgenommen. Dazu kann eine 50ml-serologische Pipette bzw. eine 5ml-Kolbenhubpipette entnommen werden.

Daraufhin wird die Mikroorganismen-Fraktion mit einer 5ml-Kolbenhubpipette unter vorsichtigem Drehen des Zentrifugengefäßes entlang seiner Längsachse in ein separates Gefäß überführt. Die dabei an der Gefäßwandung haftenden Mikroorganismen werden mit einer 10µl-Impföse mechanisch abgelöst und in der Mikroorganismen-Fraktion resuspendiert. Während der Abnahme der Mikroorganismen-Fraktion kann das Zentrifugengefäß geneigt werden, um ein versehentliches Aufziehen von Restbestandteilen, die unter der Mikroorganismen-Fraktion im Zentrifugengefäß enthalten sind, zu verhindern. Das Volumen der abgenommenen Mikroorganismen-Fraktion kann mittels entsprechender Pipetten bestimmt werden, um später die exakte Zählzahl ausgehend von Proben zu bestimmen.

Zur Bestimmung der Zellzahl sowie des Zellstatus der Mikroorganismen in der Mikroorganismen-Fraktion kann eine Probe aus dieser entnommen werden und bis zur Stufe 10⁻⁵ verdünnt werden. Konkret kann dies in 1,5 ml Reaktionsgefäßen erfolgen, wobei zur Verdünnung eine ¼-Ringerlösung (autoklaviert, steril filtriert 0,2 µm) verwendet wird.

Von der verdünnten Lösung können 195 µl in ein 5 ml Probenröhrchen überführt werden, bevor jeweils 2,5 µl SYBR Green I-Arbeitslösung und Propiumiodid-Arbeitslösung zugegeben werden. Dabei handelt es sich um Fluoreszenzfarbstoffe.

Nach einer Inkubation von 10 Minuten bei einer Temperatur von 37°C in Dunkelheit können die Proben an einem Durchflusszytometer mit entsprechenden Einstellungen gemessen werden.

Vorliegend kann dazu ein Laser mit einer Wellenlänge von 488 nm verwendet werden. Auf diese Weise kann die Zellzahl und der Zellstatus der Probe ermittelt werden, woraus auf die Gesamtzellzahl der gesamten Mikroorganismen-Fraktion rückgeschlossen werden kann.

Schließlich kann die abgenommene Mikroorganismen-Fraktion nach Abschluss der durchflusszytometrischen Messungen mit einer Abkühlrate von -1°C pro Minute auf eine Temperatur von -80°C tiefgefroren werden. Da die Hilfssubstanz mit einem präformierten Dichtegradienten lodixanol enthält, erfolgt das Einfrieren ohne Beschädigung der Mikroorganismen, da lodixanol gleichzeitig als Kryoschutzstoff wirkt.

Mit dem vorstehend beschriebenen Verfahren ist es möglich, eine Mikroorganismen-Fraktion aus der Stuhlprobe eines Patienten zu extrahieren und anschließend über einen längeren Zeitraum zu lagern, bis sie dem gleichen Patienten oder einem anderen Patienten wiedereingesetzt wird, insbesondere nachdem zwischenzeitlich eine Behandlung mit Antibiotika stattgefunden hat.

## Patentansprüche

1. Verfahren zur Extraktion und Konservierung von Mikroorganismen aus einer Stuhlprobe eines Lebewesens, insbesondere eines Menschen, umfassend die folgenden Schritte:
- Trennen einer Stuhlprobe in mehrere Fraktionen, wobei sich eine Mikroorganismen-Fraktion ausbildet, welche Mikroorganismen des Darmmikrobioms des Lebewesens enthält;
- Einfrieren der Mikroorganismen-Fraktion zur Konservierung derart, dass die Mikroorganismen zumindest teilweise in einer lebensfähigen Form erhalten bleiben.

2. Verfahren des Anspruch 1, **dadurch gekennzeichnet, dass** das Trennen der Stuhlprobe in mehrere Fraktionen mittels Dichtegradientenzentrifugation erfolgt.

3. Verfahren nach Anspruch 2, **dadurch gekennzeichnet, dass** das vor dem Trennen der Stuhlprobe in mehrere Fraktionen eine Hilfssubstanz mit einem präformierten Dichtegradienten in einem Behälter hergestellt wird.

4. Verfahren nach Anspruch 3, **dadurch gekennzeichnet, dass** die Hilfssubstanz ein chemisch inertes Mittel, insbesondere Cäsiumchlorid, Percoll, Saccharose und/oder lodixanol enthält.

5. Verfahren nach Anspruch 4, **dadurch gekennzeichnet, dass** die Hilfssubstanz lodixanol enthält, welches mit Ringerlösung verdünnt ist und/oder dass die Konzentration an lodixanol in der Hilfssubstanz mindestens 10 Gewichts-%, insbesondere mindestens 20 Gewichts-%, und/oder höchstens 40 Gewichts-%, insbesondere höchstens 30 Gewichts-%, bevorzugt 25 Gewichts-% beträgt, und/oder dassdie Hilfssubstanz eingefroren und anschließend aufgetaut wird, um einen präformierten Dichtegradienten in der Hilfssubstanz zu erzeugen, wobei, bevorzugt die Hilfssubstanz bis zu einer Temperatur von mindestens -15°C, insbesondere von -20°C eingefroren wird und/oder anschließend bei einer Umgebungstemperatur von mindestens 2°C, insbesondere von 4°C aufgetaut wird.

6. Verfahren nach einem der vorherigen Ansprüche, **dadurch gekennzeichnet, dass** vor dem Trennen der Stuhlprobe in mehrere Fraktionen diese insbesondere mit Ringerlösung verdünnt und/oder homogenisiert wird, so dass eine Stuhlsuspension entsteht.

7. Verfahren nach Anspruch 6, **dadurch gekennzeichnet, dass** zur Dichtegradientenzentrifugation die Stuhlsuspension gemeinsam mit der Hilfssubstanz mit dem präformierten Dichtegradienten in einem Behälter zentrifugiert wird, so dass sich in dem Behälter verschiedene schichtförmige Fraktionen ausbilden, wobei, insbesondere nach dem Zentrifugieren die Mikroorganismen-Fraktion von den übrigen Fraktionen getrennt wird.

8. Verfahren nach einem der vorherigen Ansprüche, **dadurch gekennzeichnet, dass** die Mikroorganismen-Fraktion auf eine Temperatur unterhalb von -50°C, insbesondere unterhalb von -60°C, bevorzugt von -80°C eingefroren wird, wobei, bevorzugtdas Einfrieren mit einer Abkühlrate von mindestens -0,1°C pro Minute, insbesondere von mindestens -0,5°C pro Minute, und/oder von höchstens -5°C pro Minute, insbesondere von höchstens -2,5°C pro Minute, bevorzugt von -1°C pro Minute erfolgt.

9. Verfahren nach einem der vorherigen Ansprüche, **dadurch gekennzeichnet, dass** vor dem Einfrieren der Mikroorganismen-Fraktion die Zellzahl und/oder der Zellstatus der Mikroorganismen in der Mikroorganismen-Fraktion ermittelt wird.

10. Verfahren nach einem der vorherigen Ansprüche, **dadurch gekennzeichnet, dass** die Bestimmung der Zellzahl und/oder des Zellstatus mittels Durchflusszytometrie erfolgt.

11. Verfahren nach Anspruch 10, **dadurch gekennzeichnet, dass** zur Durchführung der Durchflusszytometrie eine Probe der Mikroorganismen-Fraktion bis zur Verdünnungsstufe 10⁻⁵ verdünnt wird, wobei insbesondere die Verdünnung mit Ringerlösung erfolgt.

12. Verfahren nach Anspruch 11, **dadurch gekennzeichnet, dass** nach der Verdünnung eine Arbeitslösung, welche Fluoreszenzfarbstoffe enthält, der Probe zugeführt wird, wobei, insbesondere, die Arbeitslösung die Fluoreszenzfarbstoffe SYBR Green I und Propidiumiodid enthält.

13. Verfahren nach Anspruch 11 oder 12, **dadurch gekennzeichnet, dass** bei der Druchflusszytometrie die Vorwärtsstreuung und die Seitwärtsstreuung von einem auf die Probe auftreffenden Laserstrahl erfasst wird, um die Zellzahl und den Zellstatus der Mikroorganismen in der Probe zu ermitteln.
